# EUROPÄISCHE PATENTSCHRIFT

(11) **EP 2 944 331 B1**
(45) Veröffentlichungstag und Bekanntmachung des Hinweises auf die Patenterteilung: **02.10.2019**
(21) Anmeldenummer: 14167892.0
(22) Anmeldetag: 12.05.2014
(51) Int. Cl.: A61L 27/54, A61L 27/56, A61L 29/14, A61L 29/16, A61L 31/14, A61L 31/16

(54) **Implantierbarer Formkörper**
Implantable moulded part
Corps moulé implantable

(43) Veröffentlichungstag der Anmeldung: 18.11.2015
(73) Patentinhaber: B. Braun Melsungen AG, 34212 Melsungen (DE)
(72) Erfinder: Calisse, Jorge, 12203 Berlin (DE); Körber, Martin, 10367 Berlin (DE); Boxberger, Michael, 12103 Berlin (DE); Stolle, Andreas, 12101 Berlin (DE)
(74) Vertreter: Patentanwälte Ruff, Wilhelm, Beier, Dauster & Partner mbB

(56) Entgegenhaltungen:
- EP-B1- 1 933 893
- WO-A1-2009/014692
- DE-U1-202011 001 927
- US-A1- 2003 055 407
- US-A1- 2011 166 547
- US-A1- 2011 237 687

## Beschreibung

Die Erfindung betrifft in erster Linie ein Medizinprodukt, das mindestens einen Grundkörper in Form eines Ballonkatheters umfasst.

Wichtige Medizinprodukte kann man beispielsweise dahingehend unterscheiden, ob sie lediglich vorübergehend für einen Kontakt mit dem menschlichen Körper vorgesehen sind oder ob sie auf Dauer, oder doch zumindest für einen längeren Zeitraum, am oder im menschlichen Körper verbleiben sollen. Im ersteren Fall spricht man dann häufig auch von nichtimplantierbaren Medizinprodukten, im zweiten Fall von implantierbaren Medizinprodukten oder kurz von Implantaten. Alle diese Medizinprodukte sind häufig aus Metallen oder auch aus Polymeren (Kunststoffen) gefertigt.

Medizinprodukte, die sich nur vorübergehend im Körper befinden, sind beispielsweise Katheter. Dauerhaft im Körper eingebrachte Medizinprodukte (Implantate) sind beispielsweise künstliche Hüft-, Knie- und Schultergelenke, Dentalimplantate und Stents.

In der Gefäßmedizin werden dabei die sogenannten Stents als medizinische Implantate in Blutgefäße, speziell die Herzkranzgefäße eingebracht, um diese, in der Regel nach deren Aufdehnung offen zu halten und einen erneuten Gefäßverschluss zu verhindern. Das Einbringen der Gefäßstents erfolgt dabei mit Hilfe geeigneter Gefäßkatheter, insbesondere Herzkatheter. Dabei handelt es sich häufig um sogenannte Ballonkatheter, bei denen die Gefäßverengung mit Hilfe eines Ballons expandiert wird. Wie bereits angesprochen, wird dann ein Stent implantiert, um eine erneute Verengung des Gefäßes, insbesondere des Herzkranzgefäßes zu unterbinden.

Aus dem Stand der Technik ist es bereits bekannt, Medizinprodukte an ihren äußeren und inneren (Ober-)Flächen mit Beschichtungen zu versehen. Solche Beschichtungen dienen den unterschiedlichsten Zwecken. Unter anderem sind Medizinprodukte auch mit Medikamenten beschichtet, beispielsweise mit Antibiotika, um Infektionen zu verhindern oder einzudämmen.

In diesem Zusammenhang gibt es in der Angiologie, d. h. bei der Diagnose und Behandlung von Gefäßerkrankungen, auch bereits Stents, die mit wachstumshemmenden Medikamenten beschichtet sind. Solche Stents sind dazu vorgesehen, die Entwicklung einer sogenannten Restenose, d. h. eine Wiederverengung der Herzkranzarterie, nach einer Aufweitung dieser Arterie und/oder der Implantation eines Stents zu verhindern. Dabei besteht der Stent, der das entsprechende Arzneimittel freisetzt, aus einem konventionellen Stent mit einer Beschichtung, bspw. einer Polymerbeschichtung, die den antiproliferativen Wirkstoff enthält.

Das Thema derartiger medikamentenbeschichteter Stents ist beispielsweise in der EP-B1-1933893 beschrieben. Das dort offenbarte Implantat ist mit mindestens zwei Arzneimitteln beschichtet, wobei es sich bei dem ersten Arzneimittel um Rapamycin oder ein Analogon davon handelt, und bei dem zweiten Arzneimittel um Carvedilol, 17β-Estradiolvalerat oder Probucol. Gemäß dieser Druckschrift wird das zunächst unbeschichtete Implantat entweder nacheinander oder gleichzeitig mit dem ersten und dem zweiten Arzneimittel beschichtet, insbesondere sprühbeschichtet. Dies führt zu mindestens einer geschlossenen Schicht aus den genannten Materialien auf dem Implantat.

In der US 2011/237687 A1 ist die Einbringung eines Wirkstoffs in Polymere beschrieben. Hierbei wird eine Funktion zwischen der Härte des Polymers und der Wirkstoffmenge hergestellt. Neben einer Vielzahl weiterer Werkstoffe sind auch Rapamycin und Paclitaxel als Wirkstoffe erwähnt.

Die DE 20 2011 001 927 U1 beschreibt einen Stent aus einem (natürlichen) resorbierbaren Material, welcher Paclitaxel und/oder Probucol enthalten kann.

Ein Ballonkatheter ist in der US 2011/166547 A1 beschrieben. Bei diesem können vorzugsweise Rapamycin, aber auch andere Wirkstoffe über Micellen in das umgebende Gewebe appliziert werden. Irgendwelche Mischungen zwischen Paclitaxel und Probucol kommen gemäß dieser Schrift aber nicht zum Einsatz.

In der US 2003/055407 A1 geht es um Implantate, die an ihrer Außenoberfläche sogenannte Microtubes (wahlweise aus Metall, Keramik oder Hartkohlenstoff) aufweisen. Diese Microtubes, die wahlweise in eine Polymermatrix eingebunden sein können, enthalten pharmazeutische Wirkstoffe, wobei hier unter vielen anderen Wirkstoffen auch Probucol und Paclitaxel erwähnt sind.

Die WO 2009/014692 A1 beschreibt einen Stent, bei dem ein poröses Pellet in einer Ausnehmung in der Seitenwand des Stents vorgesehen ist. Dieses Pellet trägt Wirkstoffe in den Poren, wobei hier, ebenfalls neben einer Vielzahl anderer Wirkstoffe, auch Paclitaxel, Rapamycin und Probucol erwähnt sind.

Ausgehend von dem genannten Stand der Technik stellt sich die Erfindung die Aufgabe, die Ausstattung von Medizinprodukten mit Medikamenten oder Arzneimitteln, insbesondere mit antiproliferativen oder immunsuppressiven Medikamenten oder Arzneimitteln zu verbessern. Insbesondere soll mit der Erfindung eine kontrollierte Freisetzung der entsprechenden Substanzen aus dem Medizinprodukt heraus in das entsprechende Körpergewebe hinein ermöglicht werden. Die Erfindung soll dabei gerade in der Angiologie, d. h. im Zusammenhang mit Gefäßkathetern, insbesondere Ballonkathetern und Gefäßstents Anwendung finden.

Diese Aufgabe wird zum einen gelöst durch das Medizinprodukt mit den Merkmalen des Anspruchs 1 und zum anderen durch den implantierbaren Formkörper mit den Merkmalen des Anspruchs 12. Bevorzugte Ausführungen dieses Medizinprodukts und dieses Formkörpers sind in den jeweils abhängigen Ansprüchen 2 bis 11 bzw. 13 bis 16 dargestellt.

Der Wortlaut sämtlicher Ansprüche wird hiermit durch Bezugnahme zum Inhalt dieser Beschreibung gemacht.

Durch die Erfindung wird ein Medizinprodukt der eingangs genannten Art bereitgestellt, bei dem auf mindestens einem Teil der äußeren Oberfläche des Ballons des Ballonkatheters mindestens ein, vorzugsweise mindestens zwei implantierbare Formkörper vorgesehen sind. Dabei besteht der erfindungsgemäße implantierbare Formkörper aus einer Mischung aus zwei Komponenten. Bei der ersten Komponente handelt es sich um Probucol, und bei der zweiten Komponente um mindestens einen Stoff, der aus der Gruppe bestehend aus Sirolimus (Rapamycin), Sirolimus-Analoga, nämlich Everolimus, Biolimus A9, Zotarolimus (ABT-578), Temsirolismus und Ridaforolimus (AP 23573), und Paclitaxel, ausgewählt ist.

Unter "Formkörper" soll dabei jeder Körper beliebiger Gestalt verstanden werden, der aus der genannten Mischung besteht und durch entsprechende formgebende Verfahren, wie beispielsweise Gießen, Abscheiden oder mechanische Bearbeitung eines Rohprodukts hergestellt ist.

Bei Probucol handelt es sich um eine chemische Verbindung, von der bspw. ihre antihyperlipidämische Wirkung bekannt ist. Der systematische IUPAC-Name von Probucol ist 4,4'-[propane-2,2-diylbis(thio)] bis (2,6-di-tert-butylphenol).

Bei Sirolimus handelt es sich um ein Immunsuppressivum, das auch unter dem Namen Rapamycin bekannt ist (Summenformel C₅₁H₇₉NO₁₃).

Paclitaxel ist ein Zytostatikum mit der Summenformel C₄₇H₅₁NO₁₄, das genauso wie die bereits genannten Verbindungen für den Fachmann aus dem Stand der Technik bekannt ist.

Sirolimus-Analoga sind chemische Verbindungen mit den Bezeichnungen Everolimus, Biolimus A9, Zotarolimus (ABT-578), Temsirolimus, Ridaforolimus (AP 23573) .

Erfindungsgemäß kann die Mischung, aus der der erfindungsgemäße Formkörper besteht, in amorpher Form oder auch in kristalliner Struktur vorliegen.

In Weiterbildung ist der erfindungsgemäße Formkörper dadurch gekennzeichnet, dass der Gewichtsanteil der ersten Komponente, bezogen auf das Gewicht der zweiten Komponente, 1 % bis 300 %, vorzugsweise 3 % bis 200 % beträgt. Innerhalb des zuletzt genannten Bereichs sind Gewichtsanteile der ersten Komponente, bezogen auf das Gewicht der zweiten Komponente, zwischen 50 % und 150 % weiter bevorzugt.

Bei besonders bevorzugten Ausführungsformen der Erfindung ist das Gewichtsverhältnis der ersten Komponente zur zweiten Komponente in der Mischung, aus der der Formkörper besteht, ca. 1:1. Mit anderen Worten: der Gewichtsanteil der ersten Komponente, bezogen auf das Gewicht der zweiten Komponente, beträgt ca. 100 %.

Der erfindungsgemäße Formkörper kann massiv aufgebaut sein, d. h. über sein gesamtes Inneres aus der Mischung aus den beiden genannten Komponenten bestehen. Weiter sind Ausführungen möglich, bei denen im Inneren des Formkörpers mindestens ein Hohlraum vorgesehen ist. Erfindungsgemäß bevorzugt sind auch Formkörper, bei denen die Formkörper eine schaumartige oder schwammartige Struktur besitzen. Die in solchen Formkörpern vorhandenen Poren können mit Luft oder anderen Gasen oder Gasgemischen, oder auch mit Flüssigkeiten mindestens teilweise gefüllt sein.

Weiter ist es erfindungsgemäß bevorzugt, wenn im Inneren des Formkörpers mindestens teilweise Verstärkungsstrukturen oder Verstärkungsmittel vorgesehen sind. Diese dienen in diesen Fällen in erster Linie dazu, dem Formkörper selbst eine größere mechanische Festigkeit zu geben.

Der zuletzt genannte Aspekt der Erfindung wird später noch näher erläutert.

Je nach gewünschtem Einsatzzweck können die erfindungsgemäßen Formkörper eine ganz unterschiedliche Größe besitzen. Die Abmessungen des Formkörpers werden dabei in der Regel zwischen 1 nm (Nanometer) und 1 m (Meter) liegen. Vorzugsweise betragen die Abmessungen zwischen 1 µm (Mikrometer) und 10 mm (Millimeter), insbesondere zwischen 10 µm und 1 mm.

Auch die Gestalt, d. h. die dreidimensionale Form des Formkörpers, kann, insbesondere je nach beabsichtigter Verwendung, ganz unterschiedlich ausgebildet sein. So kann es sich beispielsweise um archimedische, catalanische oder Johnson-Körper handeln.

Vorzugsweise besitzt der erfindungsgemäße Formkörper im Wesentlichen die Gestalt eines Polyeders, insbesondere eines regelmäßigen Polyeders (d. h. eines platonischen Körpers).

Als bevorzugte Beispiele sind erfindungsgemäße Formkörper zu nennen, bei denen die dreidimensionale Gestalt des Körpers ausgewählt ist aus den folgenden geometrischen Formen, nämlich: Würfel, Tetraeder, Pyramide, Prisma, Deltaeder, Zylinder, Kegel, Kugel, Paraboloid, Hyperboloid und Torus.

Zu nennen sind auch Formen, die im weitesten Sinne als eine Tropfenform beschrieben werden können, sowie allgemein Polygone mit einer Zahl der Ecken zwischen 4 und 100, sowie deren geometrisch verzerrte Analoga. Auch eine gitterförmige Gestalt der Formkörper ist möglich.

In Weiterbildung ist bei der Erfindung auf mindestens einem Teil einer Oberfläche des erfindungsgemäßen Formkörpers mindestens eine Beschichtung, insbesondere eine Beschichtung mit adhäsiven Eigenschaften vorgesehen.

Die genannte Beschichtung kann grundsätzlich verschiedenen Zwecken dienen. Zum einen kann sie vorgesehen sein, um die Stabilität, insbesondere die mechanische Stabilität des Formkörpers (von außen) zu verbessern. Um in diesem Zusammenhang die biologische Abbaubarkeit und/oder die Freisetzung von Sirolimus aus dem Formkörper nicht zu behindern, kann auch diese Beschichtung abbaubar, vorzugsweise biologisch abbaubar sein.

Zum anderen kann eine Beschichtung mit adhäsiven Eigenschaften die Anlagerung oder Verbindung des Formkörpers mit dem entsprechenden (Körper-) Gewebe bei seinem medizinischen Einsatz verbessern. Auf diese Weise kann der Formkörper seine Funktion, beispielsweise einer mechanischen Stabilisierung einer Gewebestruktur, oder seine entzündungshemmende Wirkung am entsprechenden Einsatzort besser entfalten.

Die genannte Beschichtung besteht insbesondere aus einem geeigneten Polymer, vorzugsweise einem Polymer mit adhäsiven Eigenschaften, wobei es sich insbesondere bei dem Polymer um ein biologisch abbaubares Polymer handelt. In allen Fällen können dabei auch Co-Polymere oder sogenannte Blends von Polymeren eingesetzt werden.

Als Beispiele für Polymere sind insbesondere PLA (Polylactid), PLG (Co-Polymer aus Polylactid und Polyglycolid), PCL (Poly-Caprolacton), POE (Polyolefinelastomere), PE (Polyethylen), PEG (Polyethylenglycol) und PVP (Polyvinylpyrrolidon) zu nennen.

Weiter können als Beschichtungen nicht-polymere Materialien wie Lipide, z. B. Fettsäureester, Wachse, Phospholipide und Sphingolipide, oder Materialien wie Fettsäuren, Cholesterol oder Cholesterolester eingesetzt werden.

Wie bereits erwähnt, ist es erfindungsgemäß von Vorteil, wenn der Formkörper verstärkende Mittel oder verstärkende Strukturen, insbesondere zur Erhöhung seiner mechanischen Stabilität, aufweist. Hier kann es sich beispielsweise um Strukturen handeln, die innerhalb des Formkörpers bspw. als gitterartige Verstärkungsstruktur vorgesehen sind. Es ist auch möglich, verstärkende Elemente, bspw. als Pulver, in die Mischung, aus der der Formkörper besteht, einzumischen.

Vorzugsweise sind die zur Verstärkung verwendeten Materialien biologisch abbaubar, damit sie ggf. dann zusammen mit dem Material des Formkörpers selbst nach und nach im Körper abgebaut werden. Dabei kann es sich bei den entsprechenden Materialien zum einen um biologisch abbaubare Kunststoffe handeln, für die Beispiele bereits erwähnt wurden, oder um biologisch abbaubare Metalle, wie Magnesium oder Eisen.

Bei den bisherigen Ausführungen wurde der erfindungsgemäße Formkörper als einzelnes Objekt unterschiedlicher Größe und Gestalt abgehandelt und erläutert. In Abwandlung hierzu sind jedoch auch erfindungsgemäße Ausführungen möglich, bei denen der eigentliche Formkörper in einem größeren Verbund von Formkörpern, insbesondere einem größeren Verbund identisch aufgebauter Formkörper vorliegt. Der eigentliche Formkörper selbst entsteht dann erst beim Auflösen dieses Verbunds, insbesondere durch eine Fragmentierung, z. B. indem der Verbund aus Formkörpern in kleinere Verbünde aus Formkörpern oder in einzelne Formkörper zerteilt wird. Das Zerteilen kann dabei an Verbindungspunkten oder Verbindungslinien/-flächen zwischen den einzelnen Formkörpern erfolgen.

Eine solche Auflösung des Verbunds, insbesondere Fragmentierung kann dann vorzugsweise erst am eigentlichen Einsatzort des Formkörpers bei seiner medizinischen Anwendung erfolgen. Auf diese Weise kann der Formkörper in einfacherer Weise über den Verbund an Formkörpern in den Körper eingebracht und dann dort nach der Fragmentierung als einzelner Formkörper seine Wirkung entfalten. Dies wird im Zusammenhang mit den Figuren noch näher erläutert.

Die erwähnten Verbunde aus erfindungsgemäßen Formkörpern können dabei vorzugsweise die bereits im Zusammenhang mit den Formkörpern erläuterten Abmessungen und Formen besitzen. Bei Verbunden kann die Wahl einer Gitterform bevorzugt sein. Wie bereits erwähnt, enthält ein solcher Verbund vorzugsweise eine Vielzahl einzelner Formkörper (Fragmente) identischer Abmessung und Gestalt.

Bei bevorzugten Ausführungen besitzen die Fragmente (Formkörper), die aus fragmentierbaren Verbunden hervorgehen, vorzugsweise Abmessungen zwischen 1 µm und 50.000 µm, vorzugsweise zwischen 2 µm und 1.000 µm, insbesondere zwischen 10 µm und 500 µm.

Wie bereits erwähnt umfasst die Erfindung ein Medizinprodukt, bei dem als Grundkörper des erfindungsgemäßen Medizinprodukts ein sogenannter Ballonkatheter vorgesehen ist. Dabei sind dann die erfindungsgemäßen Formkörper mindestens einem Teil der äußeren Oberfläche des Ballons zugeordnet, insbesondere auf dieser Oberfläche angeordnet.

Bei weiteren bevorzugten Ausführungsformen dieses erfindungsgemäßen Medizinprodukts ist mindestens eine Schicht oder Folie vorgesehen, die die vorhandenen Formkörper mindestens teilweise nach Art einer Umhüllung umgibt. Vorzugsweise befinden sich in diesen Fällen die Formkörper zwischen dem Grundkörper und der Schicht oder Folie.

Derartige Anordnungen haben unter anderem den Vorteil, dass die Formkörper durch die Schicht oder Folie vor ihrem direkten Einsatz im Körper gegen Beschädigung, Abrieb oder dergleichen geschützt sind.

Die umhüllende Schicht oder Folie kann vorzugsweise dünn (Dicke < 1 mm, vorzugsweise < 0,5 mm) oder porös ausgebildet sein. Auf diese Weise ist es entweder möglich, die Schicht oder Folie beim Einsatz des entsprechenden Medizinprodukts leicht zu öffnen, insbesondere aufzureißen. Oder es ist sogar möglich, die Schicht oder Folie beim Einsatz des Medizinprodukts auf dem Medizinprodukt zu belassen, bspw. in der Weise, dass zwischen dem Grundkörper und der Schicht oder Folie vorgesehene Formkörper die Schicht oder Folie durchdringen. Auch dieser Gesichtspunkt wird im Zusammenhang mit den Figuren noch näher erläutert.

Der Einsatz der beschriebenen Formkörper im Zusammenhang mit den beschriebenen Medizinprodukten kann auch auf andere Art und Weise im Körper erfolgen.

So ist es nicht nur möglich, dass während des Einsatzes des Medizinprodukts die dort vorgesehenen Formkörper auf diesem Medizinprodukt verbleiben. Es ist auch möglich, die beschriebenen Formkörper entweder als solche (oder in Form eines Verbunds) oder mit Hilfe des Medizinprodukts in den Körper, insbesondere das entsprechende Körpergewebe einzubringen.

In solchen Fällen können sich die Formkörper bspw. durch Formschluss, durch eine Verbindung, durch ein Eindringen, ein Ankleben oder auch durch eine Fixierung mit Hilfe mechanischer Spannung, im Gewebe verankern, um dort direkt ihre Wirkung zu entfalten.

Eine Adhäsion der Formkörper im Gewebe kann dabei mit Hilfe einer bereits erwähnten adhäsiven Beschichtung erfolgen.

Auf diese Weise ist es auch möglich, mit Hilfe der Formkörper getrenntes Gewebe im Körper, bspw. getrennte Gewebeschichten (Intima, Media, Adventitia) miteinander zu verbinden und Läsionen mit Hilfe der Formkörper zu schließen. Auch dies wird im Zusammenhang mit den Figuren noch näher erläutert.

Auch im Körper gewollt vorhandene Hohlräume können durch den Einsatz der beschriebenen Formkörper mechanisch stabilisiert und dadurch zumindest über bestimmte Zeiträume offen gehalten werden. In diesem Zusammenhang hervorzuheben ist die erwünschte Reduzierung von Restenose und Keloidbildung mit Hilfe des Einsatzes der erfindungsgemäßen Formkörper.

Im Ergebnis wird also der erfindungsgemäße Formkörper bzw. das erfindungsgemäße Medizinprodukt lokal, beispielsweise in oder an Gefäßwände implantiert, um geschädigte Gefäße, wie Blutgefäße sowie bspw. Hohlorgane von Menschen und Tieren, welche von Veränderungen und Destabilisierungen der Gefäßwände (z. B. Delaminierungen, Dissektionen, Plaques) betroffen sind, mechanisch zu stabilisieren. Gleichzeitig wird durch die kontrollierte Freisetzung des Wirkstoffs Sirolimus aus den applizierten Formkörpern die Heilung, insbesondere die Gefäßheilung positiv beeinflusst.

Wie bereits erläutert, können dabei sowohl die einzelnen Formkörper als auch Verbunde von Formkörpern, die bspw. an bestimmten Kontaktpunkten oder Sollbruchstellen (noch) miteinander verbunden sind, eingesetzt werden. Insbesondere ist es dabei auch möglich, dass die Formkörper (einzeln oder als Verbund) auf dem Ballon des Ballonkatheters als Grundkörper/Träger/Substrat vorgesehen sind, mit deren Hilfe sie dann an den Einsatzort im Körper gebracht und dort appliziert werden. Grundkörper/Träger/Substrat sind also eine Art Applikationshilfe für den Einsatz der beschriebenen Formkörper. Dann können ggf. direkt am Einsatzort, falls erforderlich, die Formkörper vom Grundkörper/Träger/Substrat abgelöst und in das betreffende Gewebe überführt werden, ggf. auch unter Frakturierung eines Verbundes von Formkörpern in kleinere Verbunde oder einzelne Formkörper.

Die genannten und weitere Merkmale sowie Vorteile der Erfindung ergeben sich auch aus der nun folgenden Beschreibung von Figuren in Verbindung mit den Unteransprüchen. Dabei können die einzelnen Merkmale der Erfindung für sich alleine oder in Kombination miteinander verwirklicht sein. Die im Folgenden beschriebenen Ausführungsformen dienen im Übrigen lediglich zur Erläuterung und zum besseren Verständnis der Erfindung und sind in keiner Weise beschränkend zu verstehen.

In den Zeichnungen zeigen:
- Fig. 1: ein erfindungsgemäßes Medizinprodukt in Form eines Ballonkatheters mit erfindungsgemäßen Formkörpern in schematischer Ansicht, und zwar
Fig. 1A Ballonkatheter im nicht expandierten Zustand
Fig. 1B Ballonkatheter im expandierten Zustand
Fig. 1C Schnittansicht
- Fig. 2: eine weitere Ausführungsform eines erfindungsgemäßen Medizinprodukts in Form eines Ballonkatheters mit weiteren erfindungsgemäßen Formkörpern in schematischer Ansicht, und zwar
Fig. 2A Ballonkatheter im expandierten Zustand, und
Fig. 2B in Schnittansicht.
- Fig. 3: Anwendung der vorliegenden Erfindung bei einem erfindungsgemäßen Medizinprodukt mit erfindungsgemäßen Formkörpern und zusätzlicher Folie
- Fig. 4: ein nicht erfindungsgemäßes Medizinprodukt in Form eines Dilatators mit erfindungsgemäßen Formkörpern, und
- Fig. 5: eine Anwendung der erfindungsgemäßen Formkörper zur Fixierung einer GefäßDissektion.

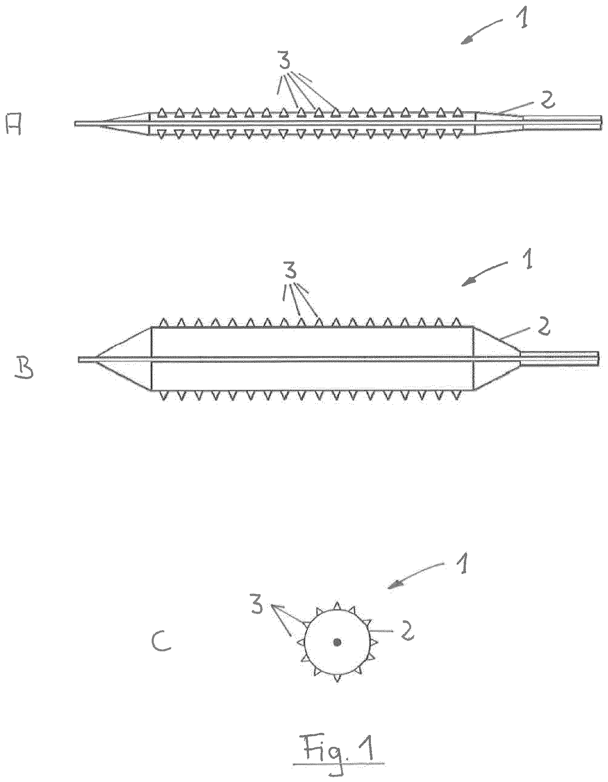

Figur 1 zeigt ein erfindungsgemäßes Medizinprodukt in Form eines Ballonkatheters 1 in schematischer Ansicht. Dabei ist aus Gründen der Übersichtlichkeit vom Ballonkatheter 1 im Wesentlichen nur der Ballon 2 dargestellt. In bekannter Weise ist dieser Ballon 2 expandierbar, bspw. um in der Angiologie verengte Gefäße, insbesondere Herzkranzgefäße in gewünschter Weise aufzuweiten.

Gemäß Figur 1 sind auf der äußeren Oberfläche des Ballons 2 Formkörper 3 angeordnet, die erfindungsgemäß aus der in den Ansprüchen definierten Mischung bestehen. Diese Formkörper 3 sind bspw. mit Hilfe einer Klebeschicht auf der äußeren Oberfläche des Ballons 2 immobilisiert. Wie erläutert, kann es sich um Formkörper 3 identischer Größe und Gestalt handeln. Es können jedoch auch verschieden geformte Formkörper unterschiedlicher Größe auf dem Ballon 2 immobilisiert sein.

Figur 1A zeigt den Ballonkatheter 1 im nicht expandierten Zustand, d. h. in dem Zustand, in dem er in üblicher Weise in das Gefäßsystem eines Patienten eingebracht wird.

Figur 1B zeigt den gleichen Ballonkatheter 1 im expandierten Zustand. Der Ballon 2 ist in üblicher Weise aufgeweitet, so dass die äußere Oberfläche des Ballons gegen die entsprechende Gefäßwand (in Figur 1B nicht dargestellt) gedrückt wird. Dadurch werden auch die Formkörper 3, die sich auf der Oberfläche des Ballons 2 befinden, gegen diese Gefäßwand gepresst und bei entsprechendem Pressdruck auch in diese Gefäßwand hinein gedrückt. Gegebenenfalls ist es auch möglich, dass die Formkörper 3 bei diesem Vorgehen in der Gefäßwand verbleiben. Dies kann bspw. durch eine entsprechende Ausgestaltung der Form der Formkörper 3 oder durch bspw. adhäsive Beschichtungen auf den Formkörpern 3 unterstützt werden.

In diesem Zusammenhang ist auch vorstellbar, dass ggf. nur ein Teil der Formkörper in die Gefäßwand gedrückt wird, und ein anderer Teil der Formkörper 3 auf der Oberfläche des Ballons 2 verbleibt.

In der beschriebenen Weise können Formkörper, die bspw. auf der Oberfläche des Ballons 2 in einem Verbund vorliegen, bei der Expansion des Ballons 2 und beim Pressen des Ballons 2 gegen die Gefäßwand, aus dem Verbund herausgelöst und vereinzelt werden.

Figur 1C zeigt den Aufbau des erfindungsgemäßen Ballonkatheters 1 in einer schematischen Schnittansicht. Dabei ist im Wesentlichen nur der Ballon 2 mit den auf seinem Außenumfang angeordneten Formkörpern 3 dargestellt.

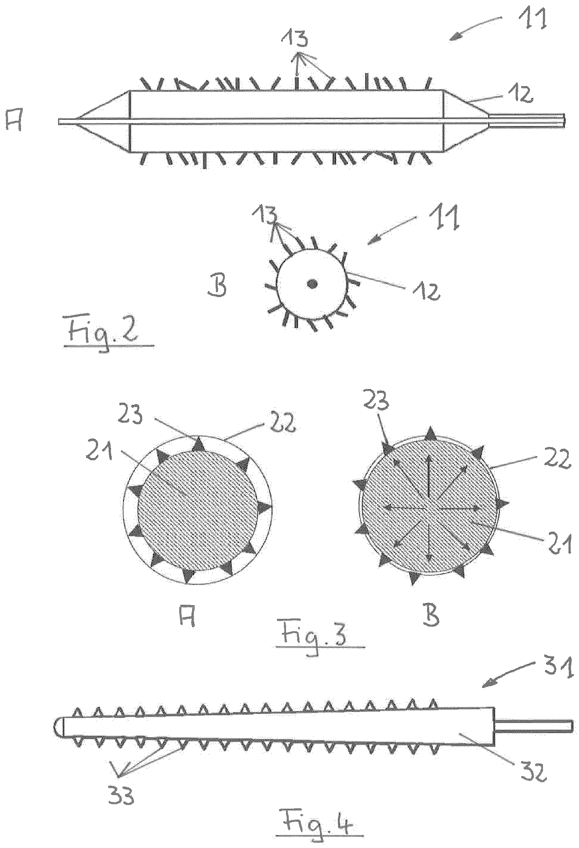

Figur 2 zeigt ein weiteres erfindungsgemäßes Medizinprodukt in Form eines Ballonkatheters 11. Hier ist in Figur 2A dieser Ballonkatheter 11 nur im expandierten Zustand dargestellt.

Auch Figur 2A zeigt den (hier bereits expandierten) Ballon 12, auf dem, wie beim Ballonkatheter 1 gemäß Figur 1, eine Vielzahl von Formkörpern 13 angeordnet und immobilisiert sind. In Figur 2A ist zeichnerisch angedeutet, dass es sich bei den Formkörpern 13 um Formkörper anderer geometrischer Gestalt handelt, als bei den Formkörpern 3 gemäß Figur 1. So ist für die Formkörper 3 gemäß Figur 1 eine pyramidale Gestalt der Formkörper angedeutet, während die Formkörper 13 gemäß Figur 2 eine stiftförmige Gestalt besitzen.

Außerdem zeigt Figur 2A, dass die Formkörper 13, im Gegensatz zu den Formkörpern 3 auf dem Ballon 2, auf dem Ballon 12 in unregelmäßiger Anordnung immobilisiert sind.

Figur 2B zeigt in vergleichbarer Weise wie Figur 1C eine schematische Schnittansicht des Ballonkatheters 11 mit Ballon 12 und Formkörpern 13.

Figur 3 erläutert die Ausführung von erfindungsgemäßen Medizinprodukten, bei denen neben einem Grundkörper und den erfindungsgemäßen Formkörpern zusätzlich eine Schicht oder Folie vorgesehen ist.

Gemäß Figur 3A befinden sich Formkörper 23 auf einem Grundkörper 21. Zusätzlich sind Grundkörper 21 und Formkörper 23 mit einer Schicht oder Folie 22, nach Art einer Hülle oder Umhüllung umschlossen. Dementsprechend befinden sich die Formkörper 23, die gemäß Figur 3A pyramidal ausgestaltet sind, mit radial nach außen abragender Spitze, zwischen dem Grundkörper 21 und der Folie 22.

Vergleicht man die Darstellung gemäß Figur 3A mit den Darstellungen gemäß Figur 1, so kann es sich bei dem Grundkörper 21 gemäß Figur 3 ohne Weiteres um den Ballon eines Ballonkatheters, gemäß Figur 1 des Ballonkatheters 1, handeln. Der Ballon entspricht somit in der Bezeichnung der Erfindung dem Grundkörper des Medizinprodukts.

Der Grundkörper 21 gemäß Figur 3A ist expandierbar ausgestaltet, wie bspw. der Ballon eines Ballonkatheters. Bei dieser Expansion werden die Formkörper 23, die sich auf dem Grundkörper 21 befinden, zunächst gegen die Folie 22 und dann durch diese Folie 22 hindurch gedrückt. Der auf diese Weise erhaltene Zustand ist in Figur 3B dargestellt.

Auf diese Weise wird erreicht, dass die Formkörper 23 auf dem Grundkörper 21 bis kurz vor der eigentlichen Anwendung des Medizinprodukts durch die (ggf. dünne und/oder poröse) Folie gegenüber äußeren Einflüssen geschützt sind. Erst nach dem Hindurchdrücken/Hindurchpressen der Formkörper 23 durch die Folie 22 hindurch entfaltet sich deren Wirkung, bspw. nachdem sie im Falle der Anwendung eines Ballonkatheters gegen und in die entsprechende Gefäßwand gepresst wurden.

Figur 4 zeigt ein weiteres nicht erfindungsgemäßes Medizinprodukt in Form eines Dilatators 31. Dieser Dilatator 31 besitzt einen konisch zulaufenden Grundkörper 32 nach Art eines Schaftes, auf dessen äußerer Oberfläche eine Vielzahl von Formkörpern 33 angeordnet und immobilisiert sind. Nachdem der Dilatator in üblicher Weise in das entsprechende Gefäß vorgeschoben wurde, wird der Dilatator aufgedehnt und zusammen mit den Formkörpern 33 gegen die innere Gefäßwandung gedrückt. Dabei können die Formkörper 33 dann die bspw. bereits im Zusammenhang mit den Figuren 1 bis 3 beschriebene Wirkung entfalten.

Auch bei der Ausführung gemäß Figur 4 ist es ohne Weiteres möglich, identische Formkörper gleicher Gestalt und Abmessungen oder verschiedene Formkörper unterschiedlicher Gestalt mit unterschiedlichen Abmessungen auf der äußeren Oberfläche des Schaftes 32 vorzusehen.

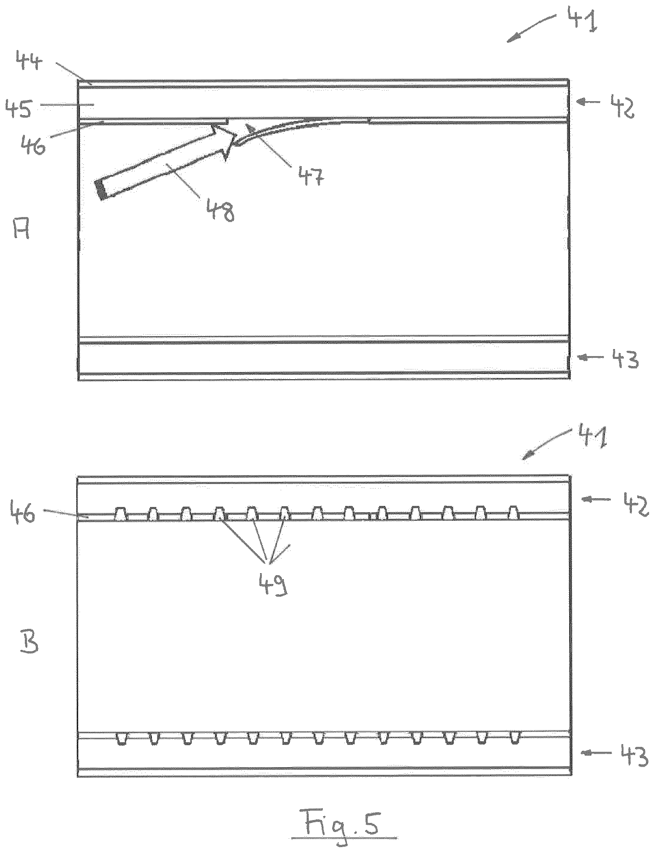

Schließlich zeigt Figur 5 eine Anwendung der Erfindung, bei der eine Dissektion oder Delaminierung eines Gefäßes durch Einsatz der erfindungsgemäßen Formkörper behandelt und fixiert wird.

Dazu ist in Figur 5A ein Blutgefäß 41 in schematischer Schnittansicht dargestellt. Dieses Blutgefäß 41 besitzt zwei Gefäßwände 42 und 43 mit deren bekanntem Schichtaufbau für solche Wandungen. Dieser Schichtaufbau ist zeichnerisch bei der Gefäßwandung 42 mit Adventitia 44, Media 45 und Intima 46 dargestellt.

Gefäßwandung 42 zeigt eine Dissektion oder Delaminierung der Intima 46 an der mit dem Bezugszeichen 47 gekennzeichneten Stelle. Der dort dargestellte Pfeil 48 zeigt den Weg, den eine aus dem Blutstrom erfolgende Einblutung nach einem solchen Intimaeinriss nehmen kann.

Der dargestellte Intimaeinriss 47 kann unter Umständen auch durch eine mechanische Belastung bei einer Ballonangioplastie hervorgerufen werden. Solche Einrisse können sogar spiralförmig über längere Abschnitte des Blutgefäßes 41 erfolgen. Nach einer Einblutung besteht konkrete Gefahr, dass das Blutgefäß geschlossen wird.

Figur 5B zeigt die Situation, nach dem der in Figur 5A dargestellte Einriss 47 unter Einsatz eines erfindungsgemäßen Medizinprodukts und der erfindungsgemäßen Formkörper behandelt ist.

Dabei wurde bspw. mit Hilfe der in den Figuren 1 und 2 beschriebenen Ballonkatheter 1 und 11 ein solcher Ballonkatheter in das Innere des Blutgefäßes 41 geführt und es wurden die entsprechenden Formkörper 49 durch Anpressen des jeweiligen Ballons in die Gefäßwandung 42 überführt. Dabei können die Formkörper 49 mechanisch und/oder adhäsiv in der Gefäßwandung 42 verankert sein.

Figur 5B zeigt dabei die Festlegung der Intima 46 über die entsprechende Länge des Blutgefäßes 41 mit Hilfe der Formkörper 49. Grob gesprochen könnte man die Funktion der Formkörper 49 in der Darstellung gemäß Figur 5B auch so beschreiben, dass die Intima 46 durch die Formkörper 49 an der Gefäßwandung 42 wieder "festgetackert" wird.

## Patentansprüche

1. Medizinprodukt umfassend mindestens einen expandierbaren Grundkörper (21), wobei es sich bei dem Grundkörper um einen Ballonkatheter handelt, **dadurch gekennzeichnet, dass** auf mindestens einem Teil der äußeren Oberfläche des Ballons mindestens ein, vorzugsweise mindestens zwei implantierbare Formkörper vorgesehen sind, wobei dieser Formkörper (3; 13; 23; 49) aus einer Mischung besteht, die als erste Komponente Probucol und als zweite Komponente mindestens einen Stoff, ausgewählt aus der Gruppe bestehend aus Sirolimus (Rapamycin), Sirolimus-Analoga, nämlich Everolimus, Biolimus A9, Zotarolimus (ABT-578), Temsirolimus und Ridaforolimus (AP 23573), und Paclitaxel, umfasst.

2. Medizinprodukt nach Anspruch 1, **dadurch gekennzeichnet, dass** eine Vielzahl von Formkörpern vorgesehen sind.

3. Medizinprodukt nach Anspruch 1 oder Anspruch 2, **dadurch gekennzeichnet, dass** im Formkörper der Gewichtsanteil der ersten Komponente, bezogen auf das Gewicht der zweiten Komponente, 1% bis 300%, vorzugsweise 3% bis 200%, insbesondere 50% bis 150% beträgt.

4. Medizinprodukt nach einem der vorhergehenden Ansprüche, **dadurch gekennzeichnet, dass** im Formkörper das Gewichtsverhältnis der ersten Komponente zur zweiten Komponente ca. 1:1 beträgt.

5. Medizinprodukt nach einem der vorhergehenden Ansprüche, **dadurch gekennzeichnet, dass** der Formkörper eine schaumartige oder schwammartige Struktur besitzt.

6. Medizinprodukt nach einem der vorhergehenden Ansprüche, **dadurch gekennzeichnet, dass** die Abmessungen des Formkörpers zwischen 1 nm und 10 mm, vorzugsweise zwischen 1 µm und 1 mm, betragen.

7. Medizinprodukt nach einem der vorhergehenden Ansprüche, **dadurch gekennzeichnet, dass** der Formkörper im Wesentlichen die Gestalt eines Polyeders, insbesondere eines regelmäßigen Polyeders besitzt.

8. Medizinprodukt nach einem der Ansprüche 1 bis 6, **dadurch gekennzeichnet, dass** die Form des Formkörpers ausgewählt ist aus den folgenden geometrischen Formen, nämlich: Würfel, Tetraeder, Pyramide, Prisma, Deltaeder, Zylinder, Kegel, Kugel, Paraboloid, Hyperboloid und Torus.

9. Medizinprodukt nach einem der vorhergehenden Ansprüche, **dadurch gekennzeichnet, dass** der Formkörper eine Beschichtung aufweist, insbesondere eine Beschichtung mit adhäsiven Eigenschaften, die auf mindestens einem Teil einer Oberfläche des Formkörpers vorgesehen ist, wobei vorzugsweise die Beschichtung aus einem Polymer, insbesondere einem biologisch abbaubaren Polymer besteht.

10. Medizinprodukt nach einem der vorhergehenden Ansprüche, weiter **gekennzeichnet durch** mindestens eine Schicht oder Folie (22), die die Formkörper mindestens teilweise nach Art einer Umhüllung umgibt, wobei vorzugsweise sich die Formkörper zwischen dem Grundkörper und der Schicht oder Folie befinden.

11. Medizinprodukt nach Anspruch 10, **dadurch gekennzeichnet, dass** es sich um eine dünne und/oder poröse Schicht oder Folie (22) handelt.

12. Implantierbarer Formkörper (3; 13; 23; 49) zur Implantation in Gefäßwände in der Angiologie, **dadurch gekennzeichnet, dass** er aus einer Mischung besteht, die als erste Komponente Probucol und als zweite Komponente mindestens einen Stoff, ausgewählt aus der Gruppe bestehend aus Sirolimus (Rapamycin), Sirolimus-Analoga, nämlich Everolimus, Biolimus A9, Zotarolimus (ABT-578), Temsirolimus und Ridaforolimus (AP 23573), und Paclitaxel, umfasst.

13. Formkörper nach Anspruch 12, **dadurch gekennzeichnet, dass** der Gewichtsanteil der ersten Komponente, bezogen auf das Gewicht der zweiten Komponente, 1% bis 300%, vorzugsweise 3% bis 200%, insbesondere 50% bis 150% beträgt, wobei vorzugsweise das Gewichtsverhältnis der ersten Komponente zur zweiten Komponente ca. 1:1 beträgt.

14. Formkörper nach Anspruch 12 oder Anspruch 13, **dadurch gekennzeichnet, dass** seine Abmessungen zwischen 1 nm und 10 mm, vorzugsweise zwischen 1 µm und 1 mm, betragen.

15. Formkörper nach einem der Ansprüche 12 bis 14, **dadurch gekennzeichnet, dass** er im Wesentlichen die Gestalt eines Polyeders, insbesondere eines regelmäßigen Polyeders besitzt, oder dass seine Form ausgewählt ist aus den folgenden geometrischen Formen, nämlich: Würfel, Tetraeder Pyramide, Prisma, Deltaeder, Zylinder, Kegel, Kugel, Paraboloid, Hyperboloid und Torus.

16. Formkörper nach einem der Ansprüche 12 bis 15, weiter **gekennzeichnet durch** eine Beschichtung, insbesondere eine Beschichtung mit adhäsiven Eigenschaften, die auf mindestens einem Teil einer Oberfläche des Formkörpers vorgesehen ist, wobei vorzugsweise die Beschichtung aus einem Polymer, insbesondere einem biologisch abbaubaren Polymer besteht.

## Claims

1. Medical product, comprising at least one expandable base body (21), the base body being a balloon catheter, **characterized in that**
on at least part of the exterior surface of the balloon are provided at least one, preferably at least two, implantable moulded parts, wherein said moulded part (3; 13; 23; 49) is composed of a mixture comprising, as a first component, probucol and, as a second component, at least one substance selected from the group consisting of sirolimus (rapamycin), sirolimus analogues, namely everolimus, biolimus A9, zotarolimus (ABT-578), temsirolimus and ridaforolimus (AP 23573), and paclitaxel.

2. Medical product according to claim 1, **characterized in that** a plurality of moulded parts are provided.

3. Medical product according to claim 1 or claim 2, **characterized in that** in the moulded part the weight proportion of the first component, based on the weight of the second component, is 1% to 300%, preferably 3% to 200%, in particular 50% to 150%.

4. Medical product according to any of the preceding claims, **characterized in that** in the moulded part the weight proportion of the first component to the second component is approximately 1:1.

5. Medical product according to any of the preceding claims, **characterized in that** the moulded part has a foam-type or sponge-type structure.

6. Medical product according to any of the preceding claims, **characterized in that** the dimensions of the moulded part are between 1 nm and 10 mm, preferably between 1 µm and 1 mm.

7. Medical product according to any of the preceding claims, **characterized in that** the moulded part has essentially the shape of a polyhedron, in particular of a regular polyhedron.

8. Medical product according to any of the claims 1 to 6, **characterized in that** the shape of the moulded part is selected from the following geometrical forms, namely: cube, tetrahedron, pyramid, prism, deltahedron, cylinder, cone, sphere, paraboloid, hyperboloid and torus.

9. Medical product according to any of the preceding claims, **characterized in that** the moulded part has a coating, in particular a coating exhibiting adhesive properties, which coating is provided on at least part of a surface of the moulded part, wherein preferably the coating is composed of a polymer, in particular a biodegradable polymer.

10. Medical product according to any of the preceding claims, further **characterized by** at least one coating or film (22) surrounding the moulded parts at least partially in the type of an envelope, wherein preferably the moulded parts are located between the base body and the coating or film.

11. Medical product according to claim 10, **characterized in that** the coating or film (22) is thin and/or porous.

12. Implantable moulded part (3; 13; 23; 49) for implantation in vascular walls in angiology, **characterized in that** the moulded part is composed of a mixture comprising, as a first component, probucol and, as a second component, at least one substance selected from the group consisting of sirolimus (rapamycin), sirolimus analogues, namely everolimus, biolimus A9, zotarolimus (ABT-578), temsirolimus and ridaforolimus (AP 23573), and paclitaxel.

13. Moulded part according to claim 12, **characterized in that** the weight proportion of the first component, based on the weight of the second component, is 1% to 300%, preferably 3% to 200%, in particular 50% to 150%, wherein preferably the weight proportion of the first component to the second component is approximately 1:1.

14. Moulded part according to claim 12 or claim 13, **characterized in that** the dimensions of the moulded part are between 1 nm and 10 mm, preferably between 1 µm and 1 mm.

15. Moulded part according to any of the claims 12 to 14, **characterized in that** the moulded part has essentially the shape of a polyhedron, in particular of a regular polyhedron, or that the shape of the moulded part is selected from the following geometrical forms, namely: cube, tetrahedron, pyramid, prism, deltahedron, cylinder, cone, sphere, paraboloid, hyperboloid and torus.

16. Moulded part according to any of the claims 12 to 15, further **characterized by** a coating, in particular a coating exhibiting adhesive properties, which coating is provided on at least part of a surface of the moulded part, wherein preferably the coating is composed of a polymer, in particular a biodegradable polymer.

## Revendications

1. Produit médicinal comprenant au moins un corps de base expansible (21), le corps de base étant un cathéter à ballonnet, **caractérisé en ce que** sur au moins une partie de la surface extérieure du ballonnet sont prévus au moins un, de préférence au moins deux, corps moulés implantables, ce corps moulé (3 ; 13 ; 23 ; 49) se composant d'un mélange qui comprend, en tant que premier composant, du Probucol et en tant que deuxième composant au moins une substance choisie parmi le groupe constitué de Sirolimus (Rapamycine), Sirolimus-Analoga, à savoir Everolimus, Biolimus A9, Zotarolimus (ABT-578), Temsirolimus et Ridaforolimus (AP 23573), et de Paclitaxel.

2. Produit médicinal selon la revendication 1, **caractérisé en ce qu'**une pluralité de corps moulés sont prévus.

3. Produit médicinal selon la revendication 1 ou la revendication 2, **caractérisé en ce que** la proportion en poids du premier composant par rapport au poids du deuxième composant dans le corps moulé est de 1 % à 300 %, de préférence de 3 % à 200 %, en particulier de 50 % à 150 %.

4. Produit médicinal selon l'une quelconque des revendications précédentes, **caractérisé en ce que** le rapport pondéral du premier composant au deuxième composant dans le corps moulé est d'environ 1:1.

5. Produit médicinal selon l'une quelconque des revendications précédentes, **caractérisé en ce que** le corps moulé possède une structure de type mousse ou éponge.

6. Produit médicinal selon l'une quelconque des revendications précédentes, **caractérisé en ce que** les dimensions du corps moulé sont comprises entre 1 nm et 10 mm, de préférence entre 1 µm et 1 mm.

7. Produit médicinal selon l'une quelconque des revendications précédentes, **caractérisé en ce que** le corps moulé possède essentiellement la forme d'un polyèdre, en particulier d'un polyèdre régulier.

8. Produit médicinal selon l'une quelconque des revendications 1 à 6, **caractérisé en ce que** la forme du corps moulé est choisie parmi les formes géométriques suivantes, à savoir : un cube, un tétraèdre, une pyramide, un prisme, un deltaèdre, un cylindre, un cône, une sphère, un paraboloïde, un hyperboloïde et un tore.

9. Produit médicinal selon l'une quelconque des revendications précédentes, **caractérisé en ce que** le corps moulé présente un revêtement, en particulier un revêtement ayant des propriétés d'adhésion, qui est prévu sur au moins une partie d'une surface du corps moulé, le revêtement se composant de préférence d'un polymère, en particulier d'un polymère biodégradable.

10. Produit médicinal selon l'une quelconque des revendications précédentes, **caractérisé en outre par** au moins une couche ou un film (22) qui entoure le corps moulé au moins en partie à la manière d'une enveloppe, les corps moulés se trouvant de préférence entre le corps de base et la couche ou le film.

11. Produit médicinal selon la revendication 10, **caractérisé en ce qu'**il s'agit d'une couche ou d'un film (22) mince et/ou poreu(se)(x).

12. Corps moulé implantable (3 ; 13 ; 23 ; 49) pour l'implantation dans les parois vasculaires en angiologie, **caractérisé en ce qu'**il se compose d'un mélange qui comprend, en tant que premier composant, du Probucol et en tant que deuxième composant au moins une substance choisie parmi le groupe constitué de Sirolimus (Rapamycine), Sirolimus-Analoga, à savoir Everolimus, Biolimus A9, Zotarolimus (ABT-578), Temsirolimus et Ridaforolimus (AP 23573), et de Paclitaxel.

13. Corps moulé selon la revendication 12, **caractérisé en ce que** la proportion en poids du premier composant par rapport au poids du deuxième composant est de 1 % à 300 %, de préférence de 3 % à 200 %, en particulier de 50 % à 150 %, le rapport pondéral du premier composant au deuxième composant étant de préférence d'environ 1 :1.

14. Corps moulé selon la revendication 12 ou la revendication 13, **caractérisé en ce que** ses dimensions sont comprises entre 1 nm et 10 mm, de préférence entre 1 µm et 1 mm.

15. Corps moulé selon l'une quelconque des revendications 12 à 14, **caractérisé en ce qu'**il possède essentiellement la forme d'un polyèdre, en particulier d'un polyèdre régulier, ou **en ce que** sa forme est choisie parmi les formes géométriques suivantes, à savoir : un cube, un tétraèdre, une pyramide, un prisme, un deltaèdre, un cylindre, un cône, une sphère, un paraboloïde, un hyperboloïde et un tore.

16. Corps moulé selon l'une quelconque des revendications 12 à 15, **caractérisé en outre par** un revêtement, en particulier un revêtement ayant des propriétés d'adhésion, qui est prévu sur au moins une partie d'une surface du corps moulé, le revêtement se composant de préférence d'un polymère, en particulier d'un polymère biodégradable.
